# EUROPEAN PATENT APPLICATION

(11) **EP 4 275 599 A1**
(43) Date of publication of application: **15.11.2023**
(21) Application number: 23716163.3
(22) Date of filing: 29.03.2023
(51) Int. Cl.: A61B 5/145, A61B 5/00, G16H 50/20

(54) **WEARABLE ELECTRONIC DEVICE INCLUDING PLURALITY OF BIOMETRIC SENSORS**

(30) Priority: 01.04.2022 KR 20220041210; 20.06.2022 KR 20220075025
(71) Applicant: Samsung Electronics Co., Ltd., Suwon-Si, Gyeonggi-do 16677 (KR)
(72) Inventor: The designation of the inventor has not yet been filed
(74) Representative: Gulde & Partner
(86) International application number: PCT/KR2023/004184
(87) International publication number: WO 2023/191498

(57) **Abstract**

A wearable electronic device including a plurality of biosensors is disclosed. The wearable electronic device according to various embodiments disclosed in the disclosure may include: a first biosensor configured to measure a first bio signal of a user; a first substrate including a second biosensor configured to measure a second bio signal of the user; and a second substrate disposed in a second direction being opposite to the first direction with respect to the first substrate, wherein the first biosensor includes: a light transmitter disposed on the second substrate and configured to transmit a first optical signal with respect to the user's body; a light receiver disposed on the first substrate and configured to receive a second optical signal generated through an interaction of the first optical signal with the user's body; and a controller configured to calculate the first bio signal of the user from the second optical signal received by the light receiver. Other various embodiments may be possible.

## Description

### [Technical Field]

Various embodiments of the disclosure relate to an electronic device, and more particularly, to a wearable electronic device including biosensors.

### [Background Art]

A wearable electronic device is an electronic device that is small-sized and light-weighted, and can be worn on a user's body. Since the wearable electronic device has high portability, the usability thereof can be improved. Further, since the wearable electronic device has high proximity to the user's body, it can be utilized for various purposes.

Since the wearable electronic device is worn in proximity to the user's body, it can obtain bio information from the user's body. The bio information may include, for example, information, such as heart rate, blood pressure, oxygen saturation, or blood glucose. The obtained bio information may be displayed to the user, or may be transmitted to another electronic device through a network so as to be utilized for user's healthcare. The wearable electronic device may include biosensors for measuring the bio information.

In order to manage metabolic diseases, such as diabetes, it is required to monitor the blood glucose of the patient. A biosensor for blood glucose measurement may include an invasive sensor for directly measuring glucose concentration of body fluids or a non-invasive sensor for indirectly measuring blood glucose through a band signal that passes through a skin.

### [Disclosure of Invention]

### [Technical Solution]

A wearable electronic device may include a plurality of biosensors for measuring various bio signals for effective healthcare of a wearer. A bio signal may be a heart rate, a blood glucose level, a blood pressure, a body temperature and/or a blood oxygen saturation level. However, since the wearable electronic device should have a size and a weight suitable to be worn on a human body, an internal space for mounting the plurality of biosensors may be insufficient.

Various embodiments disclosed in the disclosure can provide a wearable electronic device that is small-sized and includes a plurality of biosensors.

According to various embodiments of the disclosure, a wearable electronic device worn on a user's body to face the user's body in a first direction and configured to measure a plurality of user's bio signals includes: a first biosensor configured to measure a first bio signal of a user; a first substrate including a second biosensor configured to measure a second bio signal of the user; and a second substrate disposed in a second direction being opposite to the first direction with respect to the first substrate, wherein the first biosensor includes: a light transmitter disposed on the second substrate and configured to transmit a first optical signal with respect to the user's body; a light receiver disposed on the first substrate and configured to receive a second optical signal generated through an interaction of the first optical signal with the user's body; and a controller configured to calculate the first bio signal of the user from the second optical signal received by the light receiver.

According to other embodiments of the disclosure, a biosensor module of a wearable electronic device worn on a user's body to face the user's body in a first direction includes: a first biosensor configured to measure a first bio signal of a user; a first substrate including a second biosensor configured to measure a second bio signal of the user; and a second substrate disposed in a second direction being opposite to the first direction with respect to the first substrate, wherein the first biosensor includes: a light transmitter disposed on the second substrate and configured to transmit a first optical signal with respect to the user's body; a light receiver disposed on the first substrate and configured to receive a second optical signal generated through an interaction of the first optical signal with the user's body; and a controller configured to calculate the first bio signal of the user from the second optical signal received by the light receiver.

According to various embodiments disclosed in the disclosure, since the first substrate, on which the transmitter of the first biosensor is disposed, is disposed to overlap the second substrate including the receiver of the first bio sensor and the second bio sensor, the wearable electronic device including the plurality of bio sensors can be miniaturized.

### [Brief Description of Drawings]

In relation to the description of the drawings, the same or similar reference numerals may be used for the same or similar constituent elements.
FIG. 1 is a block diagram of an electronic device in a network environment according to various embodiments.
FIG. 2 is a perspective view illustrating a wearable electronic device according to some embodiments of the disclosure.
FIG. 3 is a perspective view of a rear surface of the wearable electronic device of FIG. 2.
FIG. 4 is an exploded perspective view of the wearable electronic device of FIG. 2.
FIG. 5A is an exploded perspective view of a wearable electronic device according to various embodiments of the disclosure.
FIG. 5B is an exploded side view of a wearable electronic device according to various embodiments of the disclosure.
FIG. 6A is a perspective view illustrating a biosensor module according to various embodiments of the disclosure.
FIG. 6B is a side view illustrating a biosensor module according to various embodiments of the disclosure.
FIG. 7A is a plan view illustrating a first surface of a first substrate of a biosensor module of the disclosure.
FIG. 7B is a plan view illustrating a second surface of a first substrate of a biosensor module according to some embodiments.
FIG. 7C is a plan view illustrating a second substrate of a biosensor module of the disclosure.
FIG. 8 is a side view illustrating an operation of a biosensor module of the disclosure.
FIG. 9 is a perspective view illustrating a first substrate according to some embodiments of the disclosure.
FIG. 10A is an exploded perspective view illustrating a biosensor module according to another embodiment of the disclosure.
FIG. 10B is a side view illustrating a biosensor module according to still another embodiment of the disclosure.

### [Mode for the Invention]

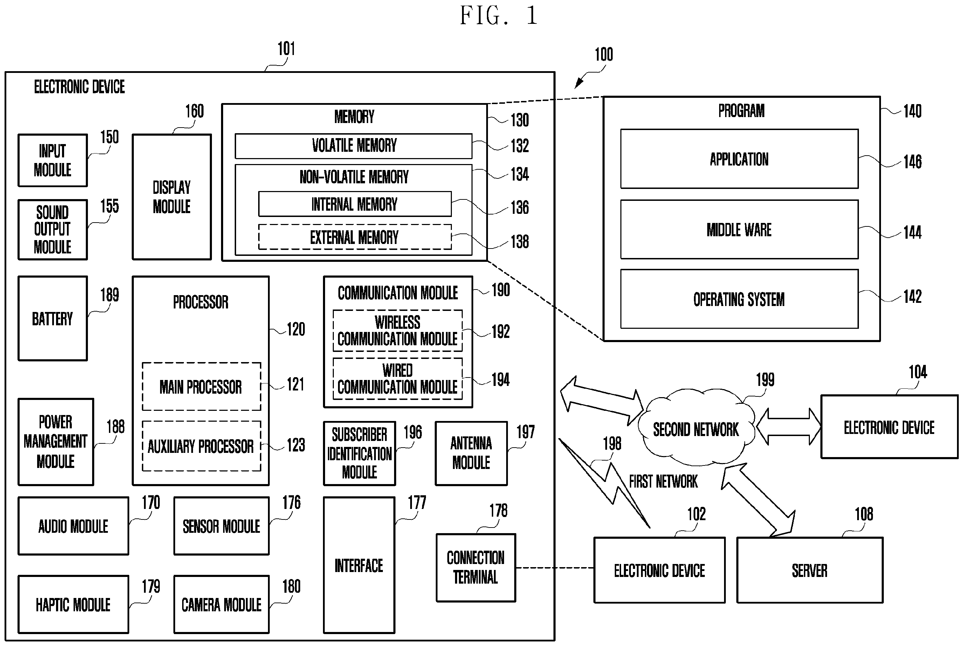

Fig. 1 is a block diagram illustrating an electronic device 101 in a network environment 100 according to various embodiments. Referring to Fig. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input 1module 150, a sound output 1module 155, a display 1module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the 11connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160). 11

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display 1module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140 may be stored in the memory 130 as software, and may include, for example, an operating system (OS) 142, middleware 144, or an application 146.

The input 1module 150 may receive a command or data to be used by another component (e.g., the processor 120) of the electronic device 101, from the outside (e.g., a user) of the electronic device 101. The input 1module 150 may include, for example, a microphone, a mouse, a keyboard, a key (e.g., a button), or a digital pen (e.g., a stylus pen).

The sound output 1module 155 may output sound signals to the outside of the electronic device 101. The sound output 1module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display 1module 160 may visually provide information to the outside (e.g., a user) of the electronic device 101. The display 1module 160 may include, for example, a display, a hologram device, or a projector and control circuitry to control a corresponding one of the display, hologram device, and projector. According to an embodiment, the display 1module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input 1module 150, or output the sound via the sound output 1module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

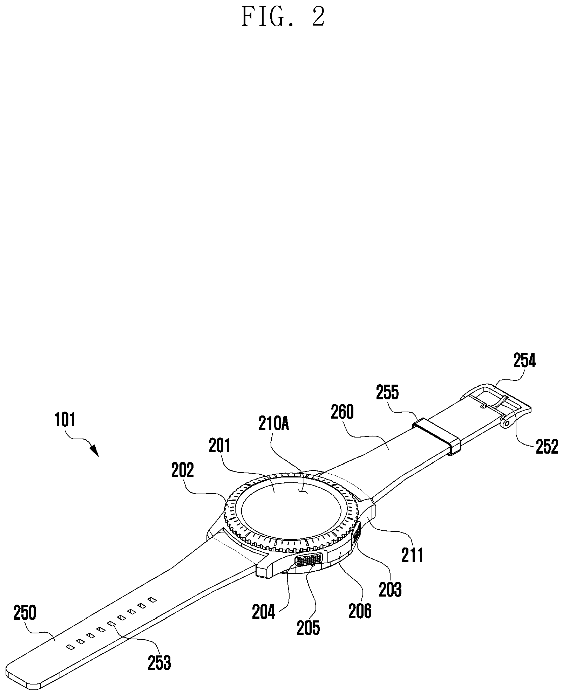

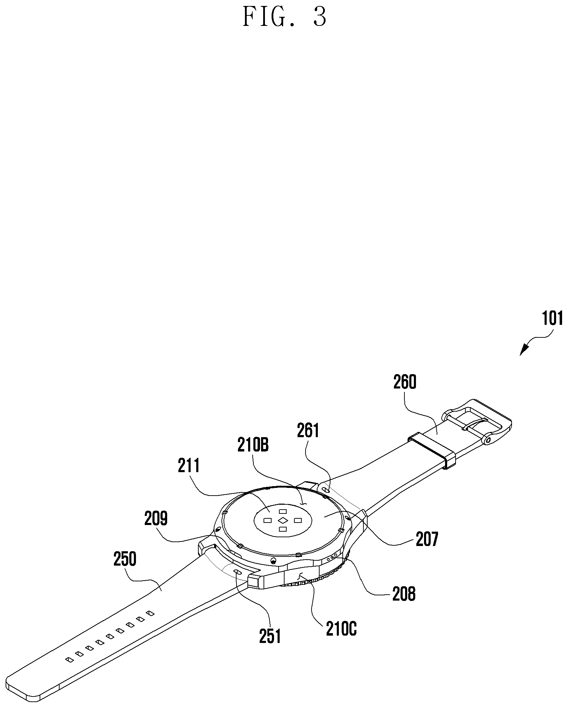

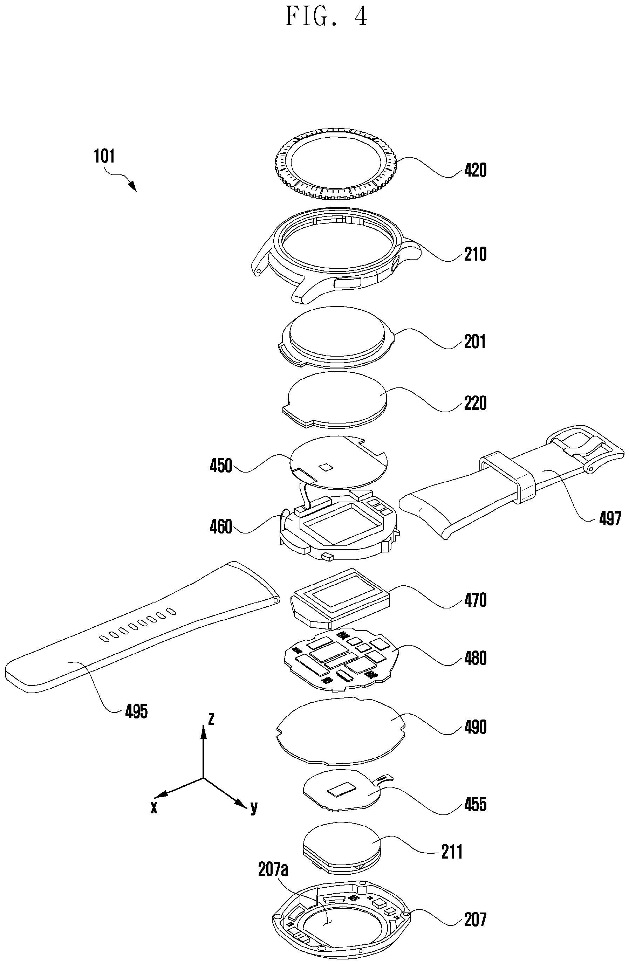

FIG. 2 is a perspective view illustrating a wearable electronic device according to some embodiments of the disclosure. FIG. 3 is a perspective view of a rear surface of the wearable electronic device of FIG. 2. FIG. 4 is an exploded perspective view of the wearable electronic device of FIG. 2.

With reference to FIGS. 2 and 3, an electronic device 101 according to an embodiment may include a housing 210 including a first surface (or front surface) 210A, a second surface (or rear surface) 210B, and a lateral surface 210C surrounding a space between the first surface 210A and the second surface 210B, and attachment members 250 and 260 connected to at least a part of the housing 210 and configured to detachably attach the electronic device 101 to a user's body part (e.g., wrist or ankle). The attachment members 250 and 260 may be a strap that wraps around the wrist to fix the electronic device 101 thereto. In another embodiment (not illustrated), the housing may be referred to as a structure that forms some of the first surface 210A, the second surface 210B, and the lateral surface 210C in FIG. 1. According to an embodiment, at least a part of the first surface 210A may be formed by a substantially transparent front plate 201 (e.g., a glass plate or polymer plate including various coating layers). The second surface 210B may be formed by a rear plate 207. The rear plate 207 may be formed by, for example, coated or colored glass, ceramic, polymer, metal (e.g., aluminum, stainless steel (STS), or magnesium), or combinations of at least two of the above materials. The lateral surface 210C may be formed by a lateral bezel structure (or "lateral member) 206 that is combined with the front plate 201 and the rear plate 207, and includes metal and/or polymer. In a certain embodiment, the rear plate 207 and the lateral bezel structure 206 may be integrally formed, and may include the same material (e.g., metal material such as aluminum). The attachment members 250 and 260 may be formed of various materials and shapes. Assembled and plural unit links may be flexibly formed by weave, leather, rubber, synthetic resin, metal, ceramic, or combinations of at least two of the above materials.

According to an embodiment, the electronic device 101 may include at least one of a display 220 (refer to FIG. 3), audio modules 205 and 208, a sensor module 211, key input devices 202, 203, and 204, and a connector hole 209. In a certain embodiment, at least one of constituent elements of the electronic device 101 (e.g., the key input devices 202, 203, and 204, connector hole 209, or sensor module 211) may be omitted, or other constituent elements may be additionally included.

The display 220 may be visually exposed, for example, through a significant part of the front plate 201. The shape of the display 220 may correspond to the shape of the front plate 201, and may be a circle, an ellipse, or a polygon. The display 220 may be disposed to be combined with or to be adjacent to a touch sensing circuit, a pressure sensor capable of measuring intensity (pressure) of a touch, and/or a fingerprint sensor.

The audio modules 205 and 208 may include a microphone hole 205 and a speaker hole 208. In the microphone hole 205, a microphone for obtaining an outside sound may be disposed, or in a certain embodiment, a plurality of microphone may be disposed so as to sense the direction of the sound. The speaker hole 208 may be used for an external speaker and a receiver for calling. In a certain embodiment, speaker holes 208 and 214 and a microphone hole 203 may be implemented as one hole, or a speaker (e.g., piezo-electric speaker) may be included without the speaker holes 208 and 214.

The sensor module 211 may generate an electrical signal or a data value corresponding to an internal operation state of the electronic device 101 or an external environment state. The sensor module 211 may include, for example, a biosensor module (e.g., HRM sensor, oxygen saturation sensor, and/or blood glucose sensor) disposed toward the second surface 210B of the housing 210. The electronic device 101 may further include a sensor module (not illustrated), for example, a gesture sensor, gyro sensor, barometric pressure sensor, magnetic sensor, acceleration sensor, grip sensor, color sensor, infrared (IR) sensor, biosensor, temperature sensor, humidity sensor, or illumination sensor.

The key input devices 202, 203, and 204 may include a wheel key 202 disposed on the first surface 210A of the housing 210 and being rotatable in at least one direction, and/or side key buttons 203 and 204 disposed on the lateral surface 210C of the housing 210. The wheel key 202 may have a shape corresponding to the shape of the front plate 201. In another embodiment, the electronic device 101 may not include some or all of the above-mentioned key input devices 202, 203, and 204, and the key input devices 202, 203, and 204 that are not included may be implemented in the form of a soft key and a touch key on the display 220. The connector hole 209 may accommodate a connector (e.g., USB connector) for transmitting and receiving a power and/or data to and from an external electronic device, or may include another connector hole (not illustrated) that can accommodate the connector for transmitting and receiving an audio signal to and from the external electronic device. The electronic device 101 may further include, for example, a connector cover (not illustrated) configured to cover at least a part of the connector hole 209 and to block an inflow of foreign substances against the connector hole 209.

The attachment members 250 and 260 may be detachably attached to at least a partial area of the housing 210 by using locking members 251 and 261. The attachment members 250 and 260 may include one or more of a fixing member 252, a fixing member fastening hole 253, a band guide member 254, and a band fixing ring 255.

The fixing member 252 may be constituted to fix the housing 210 and the attachment members 250 and 260 to the user's body part (e.g., wrist or ankle). The fixing member fastening hole 253 may fix the housing 210 and the attachment members 250 and 260 to the user's body part to correspond to the fixing member 252. Since the band guide member 254 is constituted to limit a movement range of the fixing member 252 when the fixing member 252 is fastened to the fastening hole 253, the attachment members 250 and 260 can be closely attached to the user's body part. The band fixing ring 255 may limit the movement range of the attachment members 250 and 260 in a state where the fixing member 252 and the fixing member fastening hole 253 are fastened to each other.

With reference to FIG. 4, the electronic device 101 may include a lateral bezel structure 210, a wheel key 420, a front plate 201, a display 220, a first antenna 450, a second antenna 455, a support member 460 (e.g., bracket), a battery 470, a printed circuit board 480, a sealing member 490, a rear plate 207, a biosensor module 211, and attachment members 495 and 497. The support member 460 may be disposed inside the electronic device 101, and may be connected to the lateral bezel structure 410, or may be integrally formed with the lateral bezel structure 410. The support member 460 may be formed of, for example, a metal material and/or a non-metal (e.g., polymer) material. The support material 460 may have one surface combined with the display 220 and the other surface combined with the printed circuit board 480. On the printed circuit board 480, a processor (processor 120 of FIG. 1), a memory (e.g., memory 130 of FIG. 1), and/or an interface (e.g., interface 177 of FIG. 1). The processor may include, for example, one or more of a central processing unit, an application processor, a graphic processing unit (GPU), a sensor processor, or a communication processor.

The memory may include, for example, a volatile memory or a nonvolatile memory. The interface may include, for example, a high definition multimedia interface (HDMI), a universal serial bus (USB) interface, an SD card interface, and/or an audio interface. For example, the interface may electrically or physically connect the electronic device 101 to an external electronic device, and may include a USB connector, an SD card / MMC connector, or an audio connector.

The battery 470 (e.g., battery 189 of FIG. 1) is a device for supplying a power to at least one constituent element, and may include, for example, a non-rechargeable primary cell, or a rechargeable secondary cell, or a fuel cell. At least a part of the battery 470 may be disposed, for example, on substantially the same plane as the plane of the printed circuit board 480. The battery 470 may be integrally disposed inside the electronic device 101, or may be detachably disposed in the electronic device 101.

The first antenna 450 may overlap the second antenna 455, and may be disposed between the circuit board 480 and the rear plate 207. The first antenna 450 may include, for example, a near field communication (NFC) antenna, a wireless charging antenna, and/or a magnetic secure transmission (MST) antenna. For example, the first antenna 450 may perform near field communication with an external device, may wirelessly transmit and receive the power required for charging, or may transmit a magnetism-based signal including a near field communication signal or payment data. In another embodiment, an antenna structure may be formed by parts of the lateral bezel structure 410 and/or the support member 460, or a combination thereof. In still another embodiment, the first antenna 450 may be disposed on the circuit board 480 in the form of a chip antenna.

The second antenna 455 may be disposed between the circuit board 480 and the rear plate 207. The second antenna 455 may include, for example, a near field communication (NFC) antenna, a wireless charging antenna, and/or a magnetic secure transmission (MST) antenna. For example, the second antenna 455 may perform near field communication with an external device, may wirelessly transmit and receive the power required for charging, or may transmit a magnetism-based signal including a near field communication signal or payment data. In another embodiment, an antenna structure may be formed by parts of the lateral bezel structure 410 and/or the rear plate 207, or a combination thereof.

The biosensor module 211 may be disposed adjacent to the rear plate 207 so that the rear plate 207 is oriented to the wearer's body. The biosensor module 211 may transmit a signal of various wavelength bands with respect to the wearer's body, and may measure various pieces of bio information (e.g., heartbeat, blood glucose, and/or oxygen saturation). The rear plate 207 may include a rear window 207a for passing the above-described signal with respect to the user's body or for transferring the signal from the user's body.

The sealing member 490 may be located between the lateral bezel structure 410 and the rear plate 207. The sealing member 490 may be constituted to block moisture and foreign substances flowing from outside into the space surrounded by the lateral bezel structure 410 and the rear plate 207.

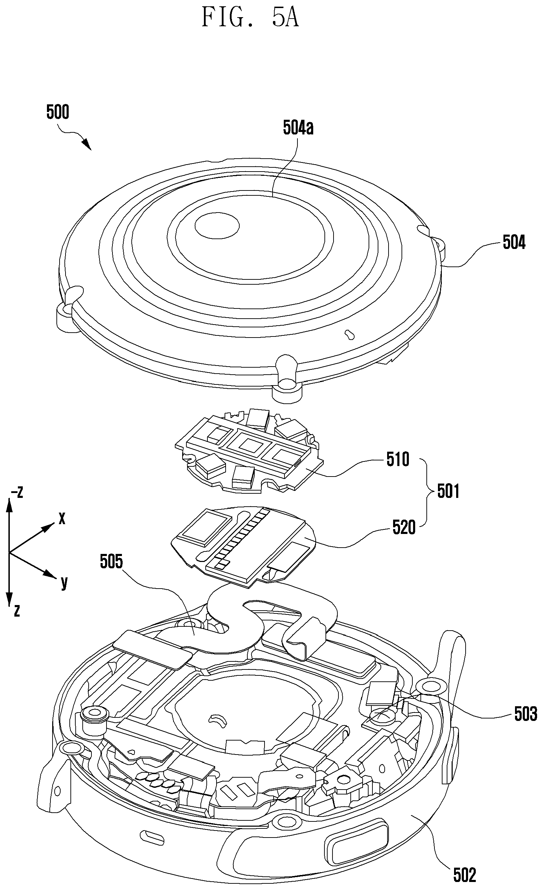

FIG. 5A is an exploded perspective view of a wearable electronic device 500 according to various embodiments of the disclosure.

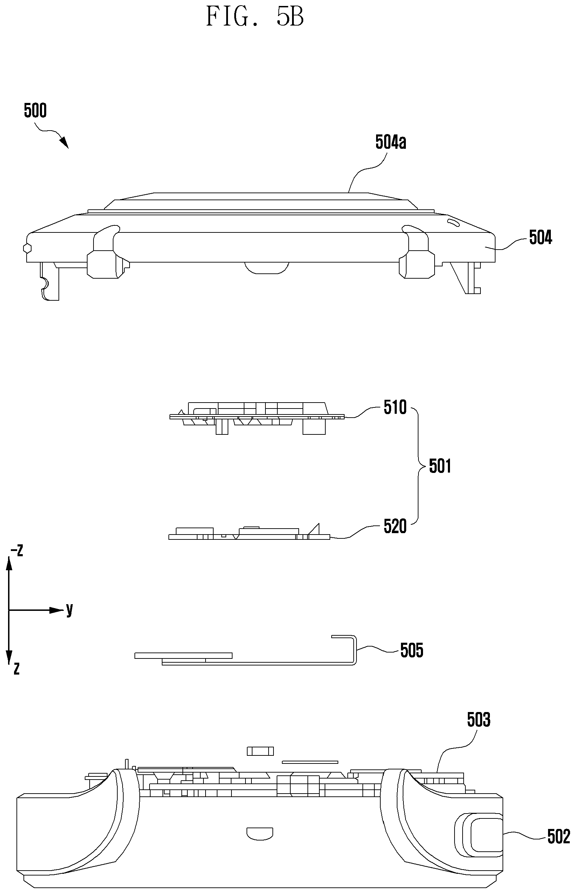

FIG. 5B is an exploded side view of a wearable electronic device 500 according to various embodiments of the disclosure.

With reference to FIGS. 5A and 5B, a wearable electronic device 500 (e.g., electronic device 101 of FIGS. 2 to 4) may include a housing 502 (e.g., housing 210 of FIG. 4), a circuit board 503 (e.g., circuit board 480 of FIG. 4), a rear plate 504 (e.g., rear plate 207 of FIG. 4), a cable 505, and a biosensor module 501.

The housing 502 and the rear plate 504 may be members for protecting constituent elements (e.g., circuit board 503) of the wearable electronic device 500, which are located therein, from being damaged due to an external impact and an inflow of foreign substances. The rear plate 504 may be an area of an external surface of the wearable electronic device 500, which faces the user's body when the user wears the wearable electronic device 500. Hereinafter, the direction (e.g., -z-axis direction) in which the rear plate 504 faces the body of the user of the wearable electronic device 500 may be referred to as "upper side". On the rear plate 504, a window may be formed, through which a signal that is emitted by the biosensor module 501 with respect to the user's body or that is received from the user's body can pass. The window 504a may include a plurality of transparent materials through which a plurality of different wavelength band signals can pass.

The biosensor module 501 may be a module that measures various bio signals from the user's body by transmitting the signal with respect to the body of the user who wears the wearable electronic device 500 and/or by receiving the signal generated from the user's body. The biosensor module 501 may include a first substrate 510 and a second substrate 520, and may include a plurality of biosensors. The detailed constitution of the biosensor module 501 will be described later. In some embodiments, the wearable electronic device 500 may include a cable 505 for connecting the biosensor module 501 and the main circuit board 503 to each other. The cable 505 may include, for example, a flexible printed circuit board (FPCB), a flexible flat cable (FFC), and/or a flexible printed cable (FPC). The biosensor module 501 may be disposed adjacent to the window 504a inside the wearable electronic device 500.

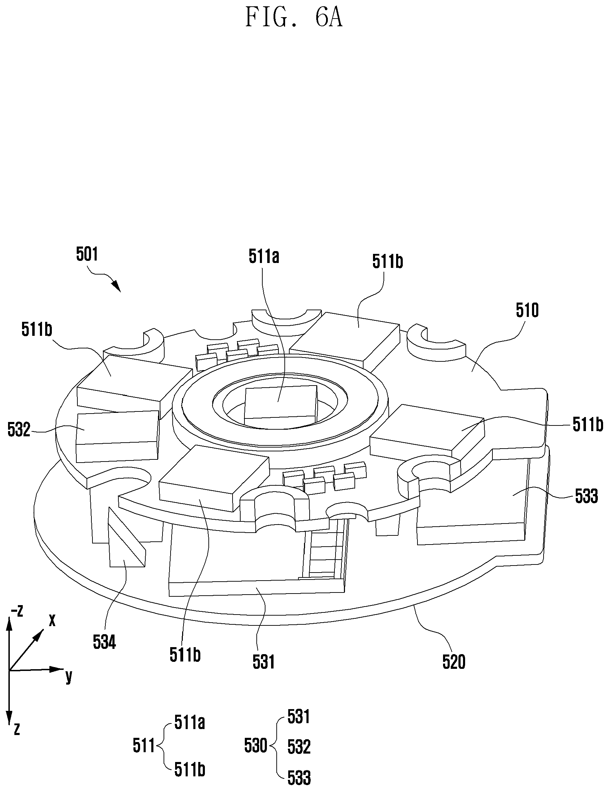

FIG. 6A is a perspective view illustrating a biosensor module 501 according to various embodiments of the disclosure.

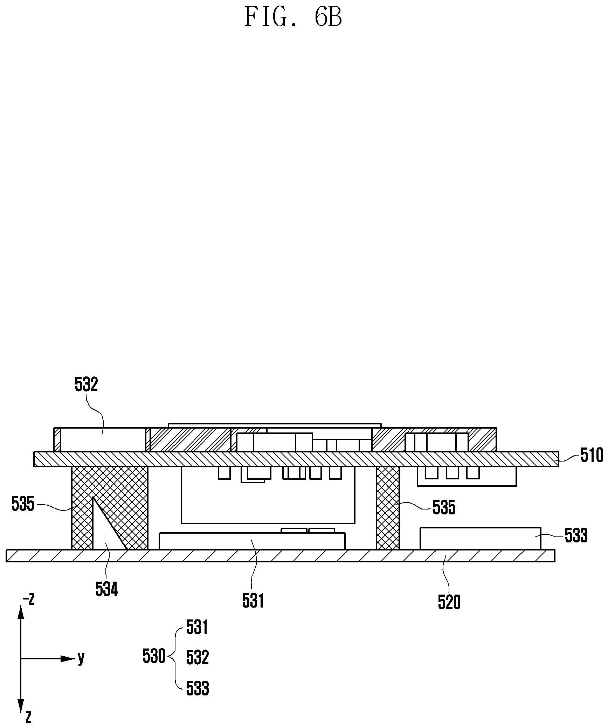

FIG. 6B is a side view illustrating a biosensor module 501 according to various embodiments of the disclosure.

With reference to FIGS. 6A and 6B, the biosensor module 501 may include the first substrate 510 and the second substrate 520. The first substrate 510 may be a substrate located relatively on the upper side based on the direction (-z-axis direction) toward the user's body, and the second substrate 520 may be a substrate located on a lower side of the first substrate 510.

The first biosensor 530 may be disposed on the first substrate 510 and the second substrate 520. The first biosensor 530 may be disposed directly on the first substrate 510 and the second substrate 520. The first biosensor 530 includes a light transmitter 531 configured to transmit a first optical signal with respect to the user's body, a light receiver 532 configured to receive a second optical signal generated from the user's body by the first optical signal, and a controller 533 configured to calculate the bio signal from the received signal of the light receiver 532. The light transmitter 531 may be disposed on the second substrate 520, and the light receiver 532 may be disposed on the first substrate 510. The light transmitter 531 may be disposed directly on the second substrate 520, and the light receiver 532 may be disposed directly on the first substrate 510. In an embodiment in which the first biosensor 530 senses the user's blood glucose, the controller 533 may be disposed on the second substrate 520. The controller 533 may be disposed directly on the second substrate 520. In some embodiments, the second biosensor 511 may be disposed on the first substrate 510. The second biosensor 511 may be disposed directly on the first substrate 510. The second biosensor 511 may include a light source 511a and a light detector 511b, and may be, for example, a sensor that measures bio information, such as the heart rate, blood pressure, and body temperature. The detailed constitutions of the first substrate 510 and the second substrate 520 will be described later.

An interposer 535 may be disposed between the first substrate 510 and the second substrate 520. The interposer 535 may be disposed directly between the first substrate 510 and the second substrate 520. The interposer 535 may be a member that electrically connects an electrical constituent element disposed on the first substrate 510, for example, the light receiver 532 of the first biosensor 530, and an electrical constituent element disposed on the second substrate 520, for example, the controller 533, to each other. The interposer 535 has the advantages of high wiring density, relatively small size, and high-bandwidth communication as compared with the cable 505 such as the flexible printed circuit (FPC).

Since the constituent elements of the first biosensor 530 are separately disposed on the first substrate 510 and the second substrate 520, the size and the volume of the biosensor module 501 including a plurality of biosensors can be reduced. Further, as described later, since the light transmitter 531 including relatively expensive constituent elements and/or the controller 533 are disposed on the second substrate, and the light receiver 532 including relatively low-priced constituent elements and occupying a small area on the substrate is disposed on the first substrate 510 together with the second biosensor 511, and since a model of the electronic device, which does not mount the first biosensor 530, but mounts only the second biosensor 511, can mount only the first substrate 510, it is possible to use the first substrate 510 as a common component in manufacturing a plurality of wearable electronic devices 500 having various sensor constitutions, and thus parts inventory management costs can be reduced.

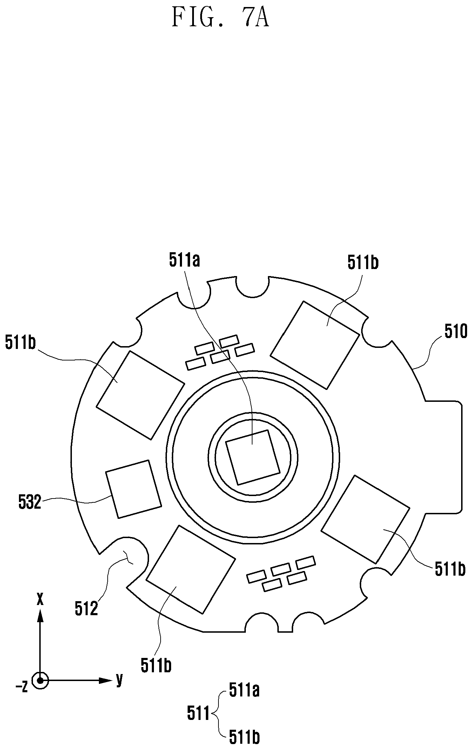

FIG. 7A is a plan view illustrating a first surface of a first substrate 510 of a biosensor module 501 of the disclosure.

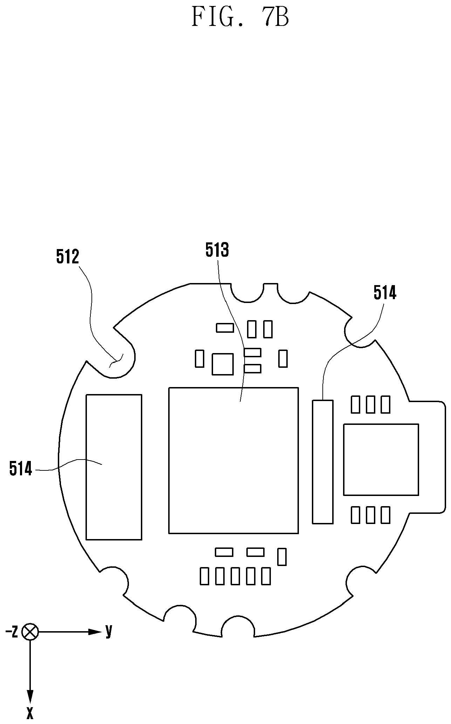

FIG. 7B is a plan view illustrating a second surface of a first substrate 510 of a biosensor module 501 according to some embodiments.

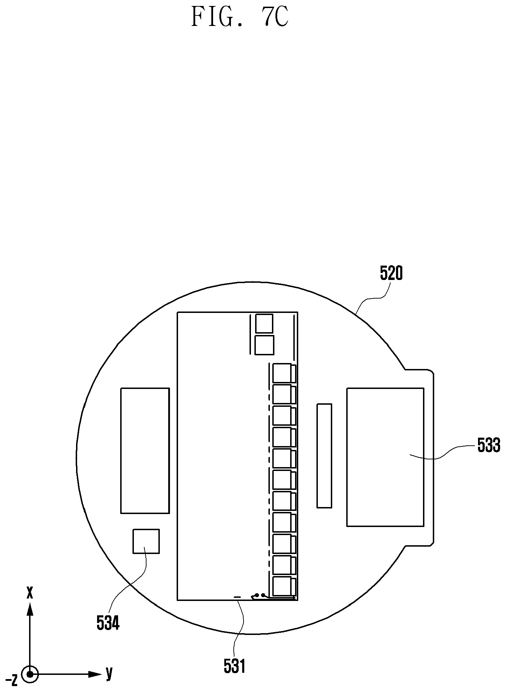

FIG. 7C is a plan view illustrating a second substrate 520 of a biosensor module 501 of the disclosure.

With reference to FIG. 7A, the light receiver 532 of the first biosensor 530 and the second biosensor 511 may be disposed on the first surface of the first substrate 510 of the biosensor module 501. The light receiver 532 of the first biosensor 530 and the second biosensor 511 may be disposed directly on the first surface of the first substrate 510 of the biosensor module 501. The second biosensor 511 may be, for example, a sensor that measures bio information, such as the heart rate, blood pressure, and body temperature. In some embodiments, the second biosensor 511 may be an optical heart rate sensor which includes a light source 511a (e.g., green LED) emitting light (e.g., green light) of a frequency band that is absorbed into the blood, and a plurality of light detectors 511b detecting a change of an absorption amount of the light emitted from a light emitter for the blood.

The light receiver 532 of the first biosensor 530 may include the light detector 511b that receives the light having a wavelength band of the second optical signal being generated from biomolecules of the user by the first optical signal being transmitted by the light transmitter 531 of the first biosensor 530, for example, a photodiode. As described later, the light receiver 532 may be constituted to convert the chronologically changed second optical signal into an electrical signal, and may measure the converted signal continuously or quasi-continuously.

The opening 512 may be formed on the first substrate 510. The opening 512 may be an opening that penetrates a partial area of the first substrate 510 so that the first optical signal being transmitted from the light transmitter 531 disposed on the second substrate 520 passes through the first substrate 510. In an embodiment, the opening 512 may be formed in an area that is adjacent to the light receiver 532 on the first substrate 510.

With reference to FIG. 7B, in some embodiments, a magnet 513 may be disposed on the second surface of the first substrate 510. The magnet 513 may be disposed directly on the second surface of the first substrate 510. The magnet 513 may be a member that fixes the wearable electronic device 500 to a charging device during charging of the wearable electronic device 500. In order to increase a fixing force of the magnet 513, it may be preferable that the magnet 513 is disposed on the first substrate 510 that is the constituent element most adjacent to the rear plate 504 inside the electronic device. In some embodiments, a first contact 514 of the interposer 535 for being connected to the interposer 535 may be disposed on the second surface of the first substrate 510.

With reference to FIG. 7C, the light transmitter 531 of the first biosensor 530 may be disposed on the second substrate 520. The light transmitter 531 of the first biosensor 530 may be disposed directly on the second substrate 520. In some embodiments, on the second substrate 520, the controller 533 configured to control the first biosensor 530 and to obtain the bio information by analyzing the electrical signal transferred from the light receiver 532 that receives the second optical signal.

In some embodiments, the light transmitter 531 may include a mid-infrared variable wavelength laser light source. Since the light transmitter 531 includes the mid-infrared variable wavelength laser light source, it is possible to obtain not only the signal generated through an interaction of the biomolecules with a single-wavelength light but also a spectrum signal generated through a reaction of the biomolecules by a plurality of wavelengths.

In some embodiments, the light transmitter 531 may include a mid-infrared laser diode, a laser resonator, a diffraction grating, a distributed Bragg reflector (DBR), am MEMS variable resonator, an optical waveguide, and/or a photonic integrated circuit on which photonics, such as the laser resonator, are two-dimensionally disposed on the substrate. Since plural photonics are monolithically disposed on the wafer by, for example, a photolithography method in the photonic integrated circuit, the volume occupied by the light transmitter 531 can be reduced as compared with a case where the plurality of optical constituent elements are individually disposed on the second substrate 520. In some embodiments, the light transmitter 531 may change the wavelength of the first optical signal generated from the light transmitter 531 by changing the wavelength of the laser light generated from the mid-infrared laser diode by the elements, such as the MEMS variable resonator, the diffraction grating, and/or the DBR, or by selecting and amplifying the laser light having different wavelengths. Since the wavelength of the first optical signal can be changed in time series, the controller can obtain the user's bio information by performing time-series analysis of the second optical signal received in the light receiver 532.

In some embodiments, a reflecting optical system 534 may be disposed on the second substrate520. The reflecting optical system 534 may be disposed directly on the second substrate 520. The reflecting optical system 534 may convert the light emitted by the photonic integrated circuit in a direction parallel to the surface of the second substrate 520 into the light in the vertical direction (-z-axis direction). The reflecting optical system 534 may include, for example, an infrared mirror and/or a prism. Since the photonic elements can be 2-dimensionally arranged by the photonic integrated circuit, the first optical signal that is modulated by the photonic elements may be emitted in parallel to the surface of the photonic integrated element. Since the reflecting optical system 534 converts the direction of the first optical signal as described above, the first optical signal can be emitted toward the user's body. In an embodiment, the reflecting optical system 534 may be disposed in an area corresponding to the opening 512 formed on the first substrate 510.

In some embodiments, a second interposer contact 524 for being connected to the interposer 535 may be disposed on the second substrate 520. The contact 524 may be disposed directly on the second substrate 520.

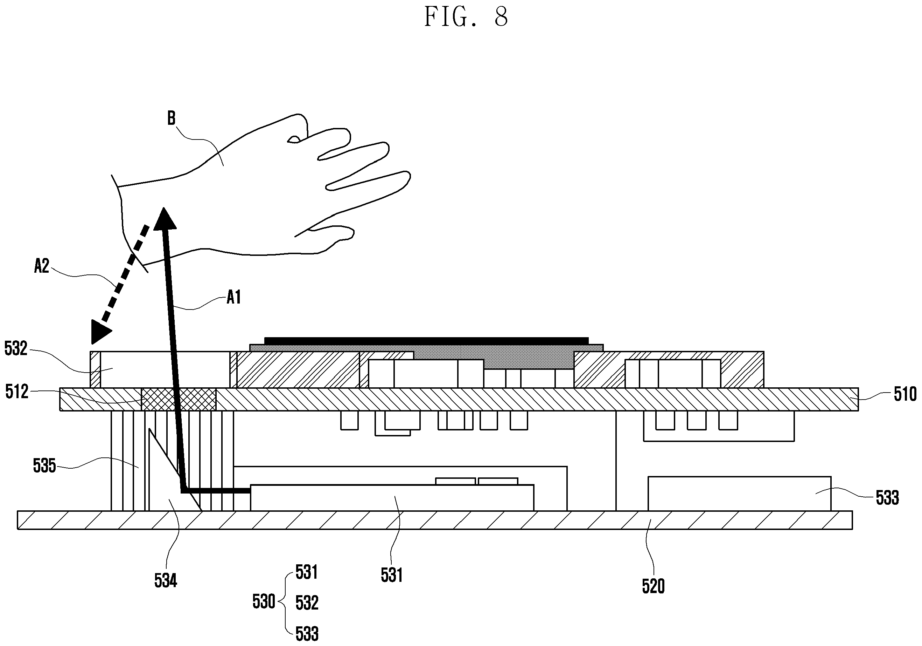

FIG. 8 is a side view illustrating an operation of a biosensor module 501 of the disclosure.

With reference to FIG. 8, the light transmitter 531 of the first biosensor 530 disposed on the second substrate 520 may transmit the first optical signal A1 with respect to the user's body B, the light receiver 532 disposed on the first substrate 510 may receive the second optical signal A2 generated through the interaction of the first optical signal A1 with the biomolecules in the user's body, and the controller 533 may analyze the electrical signal generated by the light receiver 532 through reception of the second optical signal A2 to measure the user's bio information. In some embodiments, the user's bio information being measured by the first biosensor 530 may be the blood glucose level, and the first biosensor 530 may be the non-invasive glucose measurement sensor. In some embodiments, the first optical signal A1 may include a mid-infrared laser light including a plurality of different wavelengths. The first optical signal A1 may generate the second optical signal A2 through the interaction with the biomolecules such as glucose. The second optical signal A2 may be received by the light receiver 532, and the controller 533 may measure the blood glucose concentration of the user's body by analyzing differences between interactions with the biomolecules having different wavelengths.

Since the first optical signal A1 being transmitted from the light transmitter 531 is a laser light having a low diffusion angle, it can pass through a relatively small opening 512 without a loss, and thus the light transmitter 531 may be able to be disposed on the second substrate 520 that is located on the lower side (z-axis direction) of the first substrate 510. However, since the second optical signal A2 is the optical signal being generated and radiated from the user's body through the reaction of the biomolecules of the user's body B on the laser light, the second optical signal A2 can be effectively received through the disposition of the light receiver 532 on the first substrate 510 located relatively adjacent to the upper side (-z-axis direction), that is, the user's body.

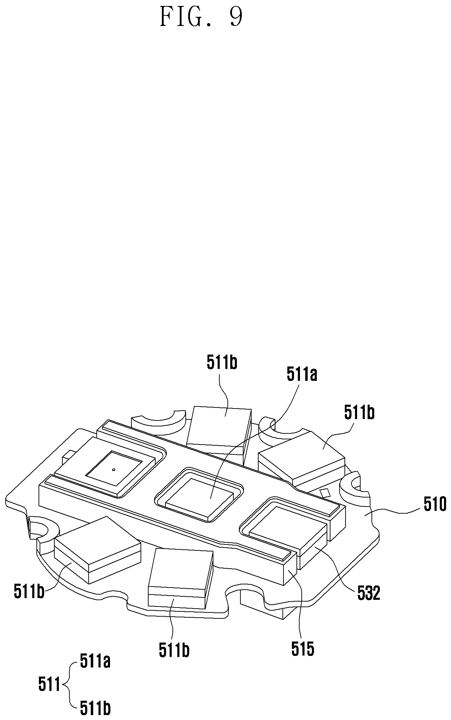

FIG. 9 is a perspective view illustrating a first substrate 510 according to some embodiments of the disclosure.

With reference to FIG. 9, the first substrate 510 of the biosensor module 501 according to some embodiments may include a shielding member 515. The shielding member 515 is a member for reducing the mutual interference between the first biosensor 530 and the second biosensor 511, and may be disposed to shield the light source 511a of the second biosensor 511 with respect to the light receiver 532 of the first biosensor 530 disposed on the first substrate 510. In some embodiments, the shielding member 515 may include a material having a high absorption rate with respect to the light of various bands, for example, a material, such as carbon black, carbon fiber, and/or carbon nanotube.

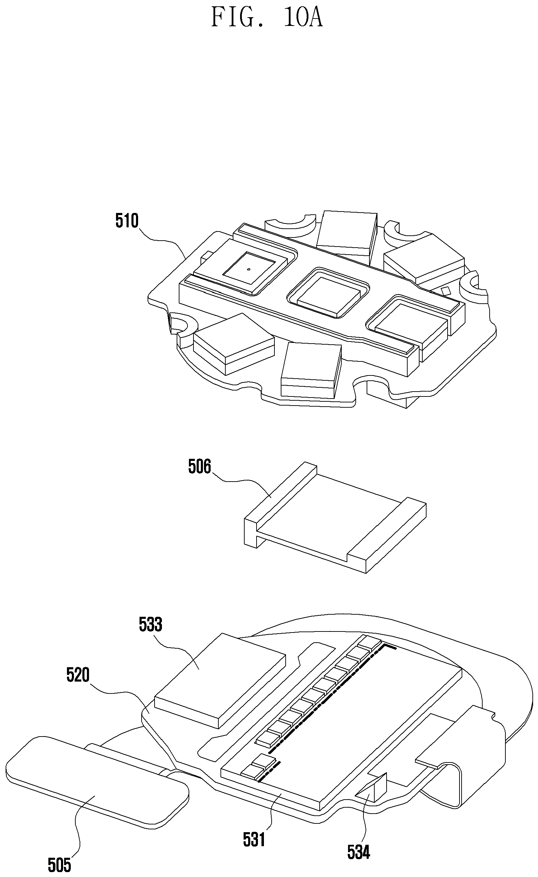

FIG. 10A is an exploded perspective view illustrating a biosensor module 501 according to another embodiment of the disclosure.

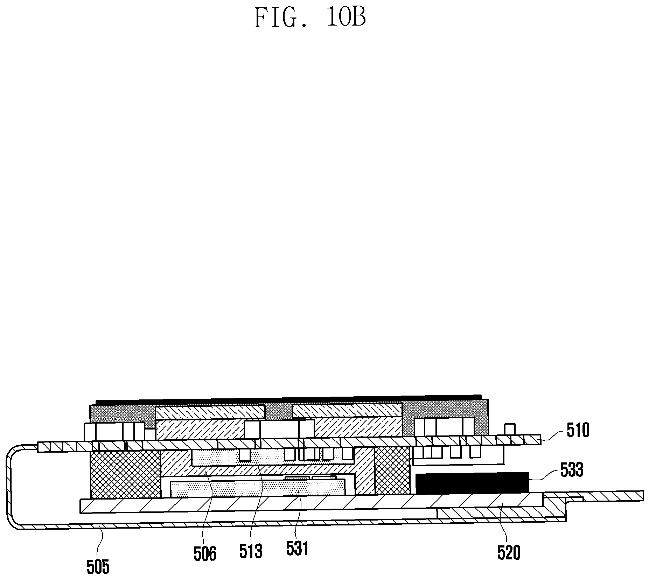

FIG. 10B is a side view illustrating a biosensor module 501 according to still another embodiment of the disclosure.

With reference to FIGS. 10A and 10B, the biosensor module 501 may include a buffer member 506 disposed between the first substrate 510 and the second substrate 520. The buffer member 506 may be disposed directly between the first substrate 510 and the second substrate 520. When an external impact is applied to the wearable electronic device 500, deflection in the lower direction (z-axis direction) may occur on the first substrate 510. Since the constituent element, such as the magnet 513, among the constituent elements disposed on the first substrate 510 has a relatively high mass, it has a high risk of warping. When an impact is applied from outside, some constituent elements of the first substrate 510 may collide with the light transmitter 531 of the second substrate 520, and there is a risk of damage to the optical constituent element of the light transmitter 531, for example, the photonic element. Accordingly, the buffer member 506 can increase the robustness of the biosensor module 501 disposed on the wearable electronic device 500.

According to various embodiments of the disclosure, a wearable electronic device 500 worn on a user's body to face the user's body in a first direction and configured to measure a plurality of user's bio signals may include: a first biosensor 530 configured to measure a first bio signal of a user; a first substrate 510 including a second biosensor 511 configured to measure a second bio signal of the user; and a second substrate 520 disposed in a second direction being opposite to the first direction with respect to the first substrate 510, wherein the first biosensor 530 includes: a light transmitter 531 disposed on the second substrate 520 and configured to transmit a first optical signal with respect to the user's body; a light receiver 532 disposed on the first substrate 510 and configured to receive a second optical signal generated through an interaction of the first optical signal with the user's body; and a controller 533 configured to calculate the first bio signal of the user from the second optical signal received by the light receiver 532. The light transmitter 531 may be disposed directly on the second substrate 520 and the light receiver 532 may be disposed directly on the first substrate 510.

In some embodiments, the first biosensor 530 may be a non-invasive glucose measurement sensor.

In some embodiments, the first optical signal may include a laser light, and the light transmitter 531 may include a mid-infrared variable wavelength laser light source. In some embodiments, the light transmitter 531 may include a photonic integrated circuit. In some embodiments, the light transmitter 531 may include a reflecting optical system 534 disposed on the second substrate 520 and configured to reflect the first optical signal being emitted from the photonic integrated circuit in a direction toward a body surface of the user. In some embodiments, the reflecting optical system 534 may include a mirror or a prism.

In some embodiments, the first substrate 510 may include an opening formed to pass the first optical signal therethrough in a partial area of the first substrate 510.

In some embodiments, the wearable electronic device may include a buffer member disposed in at least a partial area between the first substrate 510 and the second substrate 520.

In some embodiments, the wearable electronic device may include a shielding member disposed between the light receiver 532 and the second biosensor on the first substrate 510.

In some embodiments, the wearable electronic device may include at least one interposer 535 disposed between the first substrate 510 and the second substrate 520, and configured to electrically connect the light receiver 532 and the controller 533 to each other.

According to other embodiments of the disclosure, a biosensor module 501 of a wearable electronic device worn on a user's body to face the user's body in a first direction may include: a first biosensor 530 configured to measure a first bio signal of a user; a first substrate 510 including a second biosensor 511 configured to measure a second bio signal of the user; and a second substrate 520 disposed in a second direction being opposite to the first direction with respect to the first substrate 510, wherein the first biosensor 530 includes: a light transmitter 531 disposed on the second substrate 520 and configured to transmit a first optical signal with respect to the user's body; a light receiver 532 disposed on the first substrate 510 and configured to receive a second optical signal generated through an interaction of the first optical signal with the user's body; and a controller 533 configured to calculate the first bio signal of the user from the second optical signal received by the light receiver 532.

In some embodiments, the first biosensor 530 may be a non-invasive glucose measurement sensor.

In some embodiments, the first optical signal may include a laser light, and the light transmitter 531 may include a mid-infrared variable wavelength laser light source. In some embodiments, the light transmitter 531 may include a photonic integrated circuit. In some embodiments, the light transmitter 531 may include a reflecting optical system 534 disposed on the second substrate 520 and configured to reflect the first optical signal being emitted from the photonic integrated circuit in a direction toward a body surface of the user. In some embodiments, the reflecting optical system 534 may include a mirror or a prism.

In some embodiments, the first substrate 510 may include an opening formed to pass the first optical signal therethrough in a partial area of the first substrate 510.

In some embodiments, the biosensor module may include a buffer member disposed in at least a partial area between the first substrate 510 and the second substrate 520.

In some embodiments, the biosensor module may include a shielding member disposed between the light receiver 532 and the second biosensor on the first substrate 510.

In some embodiments, the biosensor module may include at least one interposer 535 disposed between the first substrate 510 and the second substrate 520, and configured to electrically connect the light receiver 532 and the controller 533 to each other.

In some embodiments, the second biosensor 511 may include a light source 511a and a light detector 511b, and wherein the light source 511a and the light detector 511b may be disposed on the first substrate 510.

In same embodiments, the first substrate 510 may comprise a first surface and a second surface, wherein the second surface faces the first direction Z and the first surface faces a second direction -Z opposite to the first direction Z, and wherein the light source 511a, the light detector 511b and the light receiver 532 may be disposed on the first surface.

In some embodiments, the first substrate 510 may include a magnet 513 disposed on the second surface.

In some embodiments, the light source 511a may be configured to emit light, and wherein the light detector 511b may be configured to detect a change of an absorption amount of the light emitted from the light source 511a, and wherein the controller 533 may be configured calculate the second bio signal of the user from the change.

Embodiments of the disclosure that are disclosed in the specification and drawings are merely for easy explanation of the technical contents of the embodiments of the disclosure and proposal of specific examples to help understanding of the disclosure, but are not intended to limit the scope of the disclosure. Accordingly, it should be construed that all changes or modifications derived based on the technical concept of the various embodiments of the disclosure are included in the scope of the various embodiments of the disclosure in addition to the embodiments disclosed herein.

## Claims

1. A wearable electronic device worn on a user's body to face the user's body in a first direction and configured to measure a plurality of user's bio signals, the wearable electronic device comprising:
a first biosensor configured to measure a first bio signal of a user;
a first substrate including a second biosensor configured to measure a second bio signal of the user; and
a second substrate disposed in a second direction being opposite to the first direction with respect to the first substrate,
wherein the first biosensor includes:
a light transmitter disposed on the second substrate and configured to transmit a first optical signal with respect to the user's body;
a light receiver disposed on the first substrate and configured to receive a second optical signal generated through an interaction of the first optical signal with the user's body; and
a controller configured to calculate the first bio signal of the user from the second optical signal received by the light receiver.

2. The wearable electronic device of claim 1, wherein the first biosensor is a non-invasive glucose measurement sensor.

3. The wearable electronic device of claim 1, wherein the first optical signal includes a laser light, and the light transmitter includes a mid-infrared variable wavelength laser light source.

4. The wearable electronic device of claim 3, wherein the light transmitter comprises a photonic integrated circuit.

5. The wearable electronic device of claim 4, wherein the light transmitter comprises a reflecting optical system disposed on the second substrate and configured to reflect the first optical signal being emitted from the photonic integrated circuit in a direction toward a body surface of the user.

6. The wearable electronic device of claim 1, comprising a buffer member disposed in at least a partial area between the first substrate and the second substrate, and
wherein the first substrate comprises an opening formed to pass the first optical signal therethrough in a partial area of the first substrate.

7. The wearable electronic device of claim 1, including a shielding member disposed between the light receiver and the second biosensor on the first substrate.

8. The wearable electronic device of claim 1, comprising at least one interposer disposed between the first substrate and the second substrate, and configured to electrically connect the light receiver and the controller to each other.

9. A biosensor module of a wearable electronic device worn on a user's body to face the user's body in a first direction, the biosensor module comprising:
a first biosensor configured to measure a first bio signal of a user;
a first substrate including a second biosensor configured to measure a second bio signal of the user; and
a second substrate disposed in a second direction being opposite to the first direction with respect to the first substrate,
wherein the first biosensor includes:
a light transmitter disposed on the second substrate and configured to transmit a first optical signal with respect to the user's body;
a light receiver disposed on the first substrate and configured to receive a second optical signal generated through an interaction of the first optical signal with the user's body; and
a controller configured to calculate the first bio signal of the user from the second optical signal received by the light receiver.

10. The biosensor module of claim 9, wherein the first biosensor is a non-invasive glucose measurement sensor.

11. The biosensor module of claim 9, wherein the first optical signal includes a laser light, and the light transmitter includes a mid-infrared variable wavelength laser light source.

12. The biosensor module of claim 11, wherein the light transmitter comprises a photonic integrated circuit.

13. The biosensor module of claim 12, wherein the light transmitter comprises a reflecting optical system disposed on the second substrate and configured to reflect the first optical signal being emitted from the photonic integrated circuit in a direction toward a body surface of the user.

14. The biosensor module of claim 9, including a shielding member disposed between the light receiver and the second biosensor on the first substrate.

15. The biosensor module of claim 9, comprising at least one interposer disposed between the first substrate and the second substrate, and configured to electrically connect the light receiver and the controller to each other.
